# EUROPEAN PATENT SPECIFICATION

(11) **EP 2 155 935 B1**
(45) Date of publication and mention of the grant of the patent: **25.01.2012**
(21) Application number: 08724379.6
(22) Date of filing: 11.04.2008
(51) Int. Cl.: D01F 1/00, D01F 6/58, D06M 11/00, B01D 39/00, D01F 6/64, D06M 11/83, B01D 39/02, D01F 6/70, D06M 13/00, D01F 1/02, D01F 11/00

(54) **FIBERS FOR DECONTAMINATION OF CHEMICAL AND BIOLOGICAL AGENTS**
FASERN ZUR DEKONTAMINIERUNG CHEMISCHER UND BIOLOGISCHER STOFFE
FIBRES POUR LA DÉCONTAMINATION D'AGENTS CHIMIQUES ET BIOLOGIQUES

(30) Priority: 11.04.2007 US 922995 P
(43) Date of publication of application: 24.02.2010
(73) Proprietor: National University of Singapore, Singapore 119077 (SG)
(72) Inventor: RAMAKRISHNA, Seeram, Singapore 119077 (SG); SUBRAMANIAN, Sundarrajan, Singapore 119077 (SG)
(74) Representative: Cabinet Plasseraud
(86) International application number: PCT/SG2008/000117
(87) International publication number: WO 2008/127200

(56) References cited:
- EP-A1- 1 857 180
- WO-A1-03/092656
- WO-A1-2005/021845
- WO-A1-2005/116140
- WO-A2-2007/095335
- US-A- 4 344 775
- US-A- 4 643 182
- US-B2- 7 160 369

## Description

### Technical Field

The present invention generally relates to fibers that can be used for decontamination of chemical and biological agents.

### Background

Threats arising from biological and chemical hazards have always existed. Such threats include warfare, incidental chemical spillage, outbreak of infectious diseases and industrial accidents involving spillage of agents etc. Chemical agents that pose potential health threats to humans and animals include toxins such as phosgene (lung damaging or choking agents), SOₓ, NOₓ, mustard agents (vesicant agents), methylphosphonothiolate (nerve agents), and hydrogen cyanide (cyanogens). Biological agents that pose potential health threats to humans and animals include from viruses such as those that cause SARS, avian flu, small pox and hemorrhagic fevers, bacteria such as those that cause plagues, cholera and diphtheria and various warfare agents such as Anthrax, Botulinum toxins and saxitoxin. In most cases, a brief immediate exposure to such agents is fatal, or can lead to permanent damage to humans and animals.

Currently, the disposal or avoidance of such hazardous agents requires both protective clothing and decontamination/detoxification agents. Protective clothing can either be impermeably or permeably adsorptive. Such protective clothing, depending on the end usage, would desirably be highly hydrophobic, highly adsorptive, flexible and possibly having certain antimicrobial activity.

Activated charcoal has been used as an adsorbent. Activated charcoal impregnated with Cu, Ag, Zn, Mo and triethylenediamine has been used as a detoxification agent in canisters for facemasks (Morrison, R.W. 2002 Overview of current collective protection filtration technology, Presented in 2002 NBC Defense collective protection conference, USA) and protective clothing. However, these masks are known to be heavy in weight and detrimentally retain moisture. Whilst gases such as phosgene, hydrogen cyanide and cyanogens chloride are easily decomposed by the impregnated metal ions, some other toxic chemicals are physisorbed and not decomposed, thereby presenting a problem in disposal after usage.

DS2 has also been used as a nerve agent decontaminant by the United States Army to wipe spillages of nerve agent. DS2 consists of 2% NaOH, 28% 2-methoxyethanol, and 70% diethylenetriamine. Whilst it is an effective nerve agent decontaminant for removal of nerve agent spillages, DS2 solution requires a reaction time of 30 minutes (i.e. low reactivity), is highly corrosive, combustible and releases toxic by-products. Furthermore, DS2 is not active against biological agents.

XE555™ resin (Ambergard Rohm & Hass Company, Philadelphia, Pennsylvania, USA) is another alternative decontaminating /detoxifying agent. However, XE555™ resin is expensive, does not possess sufficient neutralizing properties and also releases toxic gas when mixed with the sorbent.

Certain metal oxides are known to be adsorbents of hazardous chemical agents, similar to activated charcoal. Metal oxides have been shown to decontaminate/detoxify various agents such as nerve agents, blister agents, insecticide model compounds and other hazardous biological agents (Koper, O et al, Development of Reactive Topical Skin Protectants against Sulfur Mustard and Nerve Agents, J. Appl. Toxicol., 19, S59, 1999 (Stoimenov, et al, Langmuir, 2002, 18, 6679). However, a disadvantage of these known metal oxides is that they are difficult to use from a practical perspective because the metal oxides are in a form that is difficult to manipulate. For example, known metal oxides for detoxifying chemical agents are in a loose powder form which makes them difficult to contain. When removing chemical agents from the surface of an object, the metal oxide powders need to be applied onto the toxic surface and then physically removed once detoxification has been completed. This is cumbersome.

Furthermore, the free-flow of the metal oxide particles may themselves pose a potential hazard when they are freely placed on a toxic chemical agent. This is particularly the case where the metal oxide is of nano-scale or micro-scale size because the small size of the particles makes them more prone to becoming airborne and posing a potential respiratory hazard.

Furthermore, the airborne nature of the small-sized metal oxide particles renders them impractical for use in gas masks. Although the small-sized metal oxide particles can be confined to a filtered canister, the pore size of the filter must be sufficiently large to allow a sufficient flow of air. However, the large pore size of the filter means that the small-sized metal oxide particles are not able to be confined to the canister. If larger metal oxide particles are used in the canister, then there is a subsequent and exponential drop in adsorption properties of the metal oxide particles, rendering them useless as detoxification agents. Detoxifying fibers are disclosed i..a in WO 2007/95335, EP-A-1 857 180, WO 2005/21845 and WO 2005/116140.

There is a need to provide a method for effective decontamination of biological and/or chemical agents that overcomes, or at least ameliorates, one or more of the disadvantages described above.

### Summary

According to a first aspect, there is provided a nano-sized or micro-sized fiber comprising detoxifying particles, which comprise metal oxide halogen adducts.

In one embodiment, there is provided a nano-sized fiber comprising nano-sized detoxifying particles.

Advantageously, the detoxifying particles are capable of at least partially detoxifying a toxic agent.

Advantageously, the fibers, being in nano-sized or micro-sized form, can be used to manufacture materials and articles for the decontamination of surfaces contaminated with highly toxic agents such as chemical warfare ("CW"), and biological warfare ("BW") agents in addition to other toxic agents such as industrial chemicals and biological waste. Advantageously, the disclosed fiber comprising said particles is able to alter the toxic agent into a form that is at least partially, preferably completely, non-harmful to biological life. Another advantage of the disclosed fibers, being micro-sized or nano-sized, are in a form that is easy to manually handle. For example, plural fibers can be coupled together, thereby preventing the detoxifying particles from being airborne due to them being bound to the fibers.

According to a second aspect, there is provided a method for producing a fiber comprising the step of electrospinning a spinning solution having a plurality of particles suspended therein to form a nano-sized or microsized fiber comprising said particles, wherein said particles are capable of at least partially detoxifying a toxic agent.

According to a third aspect, there is provided a use of a nano-sized or micro-sized fiber comprising detoxifying particles, in the manufacture of an article for at least partially detoxifying a toxic agent.

According to a fourth aspect, there is provided a use of a nano-sized or micro-sized fiber comprising detoxifying particles, in the detoxification of a toxic agent.

According to a fifth aspect, there is provided a method of detoxifying a toxic agent comprising the steps of:
providing a nano-sized or micro-sized fiber comprising detoxifying particles; and
contacting said fiber with said toxic agent under conditions for detoxifying at least a portion of said toxic agent.

According to a sixth aspect, there is provided a nano-sized or micro-sized detoxification fiber comprising metal oxide particles capable of at least partially detoxifying a toxic agent.

According to a seventh aspect, there is provided a reactive sorbent comprising nano-sized or micro-sized fibers comprising metal oxide particles capable of at least partially detoxifying a toxic agent.

According to an eighth aspect, there is provided an article for detoxifying a toxic agent comprising a plurality of coupled nano-sized or micro-sized fibers, each of said fibers comprising detoxifying particles.

### Definitions

The following words and terms used herein shall have the meaning indicated:
The terms "toxic agent", "toxin", "toxic article" and "toxic material," are to be used interchangeably and refer to any agent that is generally harmful to biological life. Reference herein to a toxic agent is also intended to encompass CW agents, including but not limited to, toxic organophosphorus-type agents such as phosgene and derivatives and similar such art-known toxins. BW agents are also encompassed within the term toxic agent and include such agents as anthrax and botulinium toxin. In addition, unless otherwise stated, the term toxic agent as used herein is also intended to include toxic industrial chemicals, including, but not limited to, organophosphorus-type insecticides, and the like. In particular, the terms, "nerve gas," "nerve agent," "neurotoxin," and the like are intended to be equivalent, and to refer to a toxin that acts or manifests toxicity, at least in part, by disabling a component of an animal nervous system, e.g., ACHE inhibitors, as discussed supra.

The terms "detoxify", "decontaminate", "deactivate", "deactivating" and the grammatical variants thereof, in the context of this specification, when referring to toxic agents, refer to an alteration of these toxic agents to a state that is either less toxic, or nontoxic, to the functioning of biological life, particularly human and animal life. The alteration of these toxic agents may involve chemical or physical binding of the toxic agents to reduce or eliminate their toxicological activity to biological life.

The term "detoxifying particles" refers to particles that exhibit the property of detoxifying a toxic agent as described above.

The term "micro" as used herein is to be interpreted broadly to include dimensions between about 1 micron to about 500 micron.

The term "nano" as used herein is to be interpreted broadly to include dimensions less than about 1000 nm.

The term "spinning solution" is to be interpreted broadly to include any solution that is capable of being electrospun.

The term "polymer solution" is to be interpreted broadly to include any solution comprising one or more polymer, copolymer or polymer blend dissolved in a solvent and which comprises a concentration of polymers that is capable of being electrospun. Exemplary polymers include, but are not limited to, poly(vinylidenefluoride), poly(vinylidene fluoride-co-hexafluoropropylene), polyacrylonitrile, poly(acrylonitrile-co-methacrylate), poly(methylmethacrylate), polyvinylchloride, poly(vinylidenechloride-co-acrylate), polyethylene, polypropylene, nylon series such as nylon12 and nylon-4,6, aramid, polybenzimidazole, poly(vinylalcohol), cellulose, cellulose acetate, cellulose acetate butylate, poly(vinyl pyrrolidone-vinyl acetates), poly(bis-(2-methoxy-ethoxyethoxy)) phosphazene(MEEP), poly(ethylene imide), poly(ethylene succinate), poly(ethylene sulphide), poly(oxymethylene-oligo-oxyethylene), poly(propyleneoxide), poly(vinyl acetate), polyaniline, poly(ethylene terephthalate), poly(hydroxy butyrate), poly(ethylene oxide), SBS copolymer, poly(lacticacid), polypeptide, biopolymer such as protein, pitch series such as coal-tar pitch and petroleum pitch. Copolymers and blends of the above polymers may be used.

Unless specified otherwise, the terms "comprising" and "comprise", and grammatical variants thereof, are intended to represent "open" or "inclusive" language such that they include recited elements but also permit inclusion of additional, unrecited elements.

As used herein, the term "about", in the context of concentrations of components of the formulations, typically means +/- 5% of the stated value, more typically +/- 4% of the stated value, more typically +/-3% of the stated value, more typically, +/- 2% of the stated value, even more typically +/- 1% of the stated value, and even more typically +/- 0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

### Detailed Disclosure of Embodiments

Exemplary, non-limiting embodiments of a nano-sized or micro-sized fiber comprising particles capable of at least partially detoxifying a toxic agent, will now be disclosed. Also disclosed is a method of synthesizing the disclosed fibers.

### The Fibers

There is provided a nano-sized or micro-sized fiber comprising particles capable of at least partially detoxifying a toxic agent. The fiber can be woven into articles which can be used to detoxify toxic agents. For example, if the fiber is woven into a cloth, the fiber containing cloth can be used to adsorb and then detoxify the toxic agent. The toxic agent may be in the form of a liquid and/or vapor. When the toxic agent to be treated is in liquid form and is disposed on a surface, the cloth containing the fibers can be used to both adsorb the toxic agent and detoxify it.

In one embodiment, the fiber has a diameter in the micrometer range. The fiber may have a diameter of less than about 500 microns, from about 500 microns to about 400 microns, from about 400 microns to about 300 microns, from about 300 microns to about 200 microns, from about 200 microns to about 100 microns, from about 100 microns to about 10 microns, from about 10 microns to about 1 micron, about 1 micron.

In one embodiment, the fiber has a diameter in the nanometer range. The fiber may have a diameter of less than about 1000 nm, less than about 500 nm, from about 50 nm to about 500 nm and from about 100 nm to about 500 nm.

While micro-sized fibers and particles thereon will detoxify toxic agents to a certain extent, they are not as preferred as nano-sized fibers having nano-sized particles thereon. The reason being that the nano-sized fibers with nano-sized particles have a much higher surface area and therefore have more active sites on which to be exposed to the toxic agent, thereby resulting in the nano-sized fibers having a greater efficacy of action in detoxifying toxic agents relative to larger fibers.

In one embodiment, the fiber is a polymer fiber. The polymer fiber may also be hydrophobic or hydrophilic. Exemplary polymers that may be used include polyester, polysulfone, polycaprolactone, poly-halide, polyacrylates, polystyrene, polycarbonate, polyethylene oxide, polymethacrylate, polyacrylic acid, cellulose acetate, polyvinylpyrrolidone, polyvinyl acetate, polyethylene imine, polylactide, polycaprolactone, poly(glycolic acid), polyamide, polyimide, polyethylene, polyolefin, polypropylenes, polyacrylonitrile, polycarbamide, polyurethane, polymer melt, polymer blend, copolymers or terpolymers of said polymer and mixtures thereof.

Particularly useful polymers are those that have relative good adsorption properties, such as polysulfones, polyurethanes and polycarbonates as these assist in adsorbing the toxic agent. Exemplary polysulfone compounds may include polyarylsulfones, for example, bisphenol A polysulfone, polyether sulfone, polyphenyl sulfone, poly(phenoxyphenylsulfone) and mixtures thereof. Exemplary polyurethanes include poly(fluorosilicone urethane) copolymers, polyaddition products of hexamethylenediisocyanate and butane diol, polyaddition product of tolylenediisocyanate and poly(ethylene oxide). Exemplary polycarbonates include polyethylene carbonate, polybutylene carbonate, and polytrimethylene carbonate and its derivatives.

The fibers may further be coated with a material that is capable of imparting additional desired properties to the fiber to adsorb the toxic agent. For example, where the toxic agent is a nerve gas, an absorbent such as talcum powder or bentonite could also be used to coat the fibers.

In one embodiment, the particles present in said fiber are nano-sized or micro-sized. In the case where the particles are nano-sized, the particles may have an average particle size of about 10 nm to about 200 nm; about 10 nm to about 20 nm; about 10 nm to about 50 nm; about 10 nm to about 100 nm and about 50 nm to about 100 nm. In the case where the particles are micro-sized, the particles may have an average particle size of about 10 microns to about 200 microns; about 10 microns to about 20 microns; about 10 microns to about 50 microns; about 10 microns to about 100 microns and about 50 microns to about 100 microns. The size of the particles may be chosen based on the overall size of the fiber such that the average particle size of the particles is not more than the average diameter of the fiber. The particles may be regular in shape or irregular in shape. In one embodiment, the particle may be substantially symmetrical in shape. The particles may also be completely solid, partially porous, mesoporous or completely porous. The particles may exhibit a Brunauer-Emmett-Teller (BET) multi-point surface area of at least from about 40 m²/g, to about 850 m²/g or from about 100 m²/g, to about 400 m²/g. The particles may be coated or uncoated. In one embodiment, the particles have reactive functional groups attached on the surfaces thereof. These reactive functional groups may be able to aid in the detoxification of toxic agents.

The particles are metal oxide-halogen adducts such as chlorine or bromine adducts of magnesium oxide. These metal oxide-halogen adducts are particularly useful as bactericidal agents as they can destroy spores and are therefore useful against biological agents, such as anthrax. Without being bound by theory, it is thought that the metal oxide-halogen adduct is toxic to bacteria and other microbes due to the halide atom. The metal oxide-halogen adducts and bacteria are of opposite charge to each other and therefore tend to tightly bind with each other. The metal oxide-halogen adducts tend to destroy the membrane of the bacteria, thereby nullifying the bacterial activity. The metal oxide-halogen adducts can be synthesized by exposing a metal oxide, such as MgO, to a halogen gas, such as Cl₂.

The particles present within said fiber may be a mixture of different types of particles or may be of the same type of particles. The sizes of the particles present within said fiber may be substantially the same or may be substantially different from each other. The particles may be bounded to the fibers either chemically or physically.

The particles are selected from the group consisting of metal oxide halogen adducts and mixtures thereof, wherein the metal oxides, are selected from any one or more of the following group, MgO, CaO, BaO, TiO₂, SrO, ZnO, Mn₂O₃, Fe₂O₃, FeO, ZrO₂, V₂O₃, V₂O₅, CuO, NiO, Al₂O₃, CeO₂, SnO₂, Fe₃O₄, _{MnO}, Cu₂O, Nb₂O₅, InO₃, HfO₂, Cr₂O₃, Ta₂O₅, Ga₂O₃, Y₂O₃, MoO₃, Co₃O₄ SiO₂, ZnO, Ag₂O, Ag, MgO/Al₂O₃, MgO/Cl₂, MgO/I₂ and MgO/Br₂.

The particles may be coated or uncoated. In one embodiment, the metal oxide particles have at least a portion of their surfaces coated with a quantity of a second metal oxide different than the first metal oxide and selected from the group consisting of oxides of Ti, V, Fe, Cu, Ni, Co, Mn, Zn and mixtures thereof. In one embodiment, the metal oxide particle is one of MgO and CaO coated with Fe₂O₃.

### Synthesis of the Fibers

As described above, the metal oxides of the adducts may be synthesized by methods selected from the group consisting of nonhydrolytic sol-gel methods, aero-gel methods, solvothermal methods, emulsion precipitations and conventional powdering methods. An exemplary method to synthesize nano-sized metal-oxide-based materials using sol-gel processing is disclosed in United States Patent No. 6,986,818 and an exemplary aero-gel synthesis has been disclosed in Utamapanya et al., Chem. Mater., 3, 175-181.

The fibers are made in a method comprising the step of electrospinning a spinning solution having a plurality of particles suspended therein to form a nano-sized or microsized fiber comprising the particles. The apparatus used for electrospinning the fibers may comprise a nozzle for dispensing the spinning solution and a collector for collecting the spun fibers, wherein a voltage is applied between the spinning solution and the collector so that the spinning solution is charged with an opposite polarity to the collector. Either one of the spinning solution and the collector may also be charged with a voltage while the other is grounded. In one embodiment, the nozzle of the electrospinning apparatus may be charged with a voltage so as to impart the charge to the spinning solution exiting from said nozzle. A static electric field may be established between the spinning solution and the collector to thereby form a Taylor cone therebetween. In one embodiment, a positive voltage is applied to the nozzle of the electrospinning apparatus while the collector is grounded. The distance of the collector from the tip of the nozzle may be in the range of from about 5 cm to about 20 cm, from about 10 cm to about 20 cm, from about 15 cm to about 20 cm or from about 10 cm to about 15 cm. In one embodiment, the collector may be made of a material that is non reactive to the fibers or does not react adversely to the formed fibers and undermine their detoxification activity. In one embodiment, the collector is made of aluminum.

The spinning solution may comprise monomeric precursors of polymers that are adsorbent to the chemical or biological toxic agent, for example polysulfone to nerve agent.

The spinning solution also comprises said particles. In one embodiment, the particles are suspended in said spinning solution. The flow rate of the spinning solution may be controlled by a control means such as a syringe pump.

In one embodiment, a cross linking agent is added to the spinning solution before electrospinning. The addition of the cross-linking agent may serve to strengthen the electro-spun fibers. In another embodiment, the fiber produced by electrospinning is subjected to heat treatment after electrospinning. Exemplary cross linking agents include but are not limited to 1,4-butanediol diglycidyl ether (ie, bis epoxide) and glyoxal.

In one embodiment, the applied voltage for the electrospinning is from about 1 KV to about 50 KV or from about 4 KV to about 25 KV. The applied voltage may be selected in accordance to the polymer solution use. For example, when polysulfone polymer solution is used, the applied voltage may be from about 11 KV to about 13 KV. In one embodiment, the applied voltage is about 12.5 KV. In another embodiment, when the applied voltage is about 12.5 KV, the distance between the nozzle tip and the collector of the electrospinning apparatus is about 15cm.

The process of electrospinning to form the disclosed fibers may be carried out at room temperature of about 22 degrees Celsius and at a relative humidity of less than about 60%, or less than about 50%.

In one embodiment, the electrospun fibers are used to form a membrane. The membrane formed from the electrospun fibers may be dried using a drying means such as a vacuum pump.

In another embodiment, the fibers formed from the electrospinning process may be used to form a yarn. The fibers may be intertwined together to improve their strength. The fibers collected on the collector of the electrospinning apparatus may be arranged to form a random pattern or an ordered pattern.

In another embodiment, the fibers may also be directly electrospun onto a matrix material to form a composite material. Exemplary matrix materials include epoxy or other resins, ceramics, metals and the like.

In one embodiment, the disclosed fiber is capable of detoxifying toxic chemical or biological agents. The toxic chemical or biological agents may be in fluid form such as a gas, liquid or a gel. The toxic chemical or biological agents may also be in solid form such as powders that can be inhaled. The fiber may also be used in the manufacture of a material capable of absorbing and detoxifying a toxic agent. Exemplary toxic chemical agents, include agents selected from the group consisting of nerve agents and derivatives thereof, blister agents and derivatives thereof, insecticide model compounds, blood agents, acids, alcohols, compounds having an atom of P, S, N, Se, or Te, hydrocarbon compounds, and toxic metal compounds. Exemplary toxic biological agents include anthrax, plague, bacteria, fungi, viruses, rickettsiae, chlamydia, and toxins. The bacteria may include gram positive bacteria like B. globigii and B. cereus. The biological toxins may include Aflatoxins, Botulinum toxins, Clostridium perfringens toxins, Conotoxins, Ricins, Saxitoxins, Shiga toxins, Staphylococcus aureus toxins, Tetrodotoxins, Verotoxins, Microcystins (Cyanginosin), Abrins, Cholera toxins, Tetanus toxins, Trichothecene mycotoxins, Modeccins, Volkensins, Viscum Album Lectin 1, Streptococcal toxins (e. g., erythrogenic toxin and streptolysins), Pseudomonas A toxins, Diphtheria toxins, Listeria monocytogenes toxins, Bacillus anthracis toxic complexes, Francisella tularensis toxins, whooping cough pertussis toxins, Yersinia pestis toxic complexes, Yersinia enterocolytica enterotoxins, and Pasteurella toxins.
In one embodiment, the disclosed fiber is capable of the destructive adsorption of hydrocarbon compounds, both chlorinated and non-chlorinated.

### Brief Description Of Drawings

The accompanying drawings illustrate a disclosed embodiment and serve to explain the principles of the disclosed embodiment. It is to be understood, however, that the drawings are designed for purposes of illustration only, and not as a definition of the limits of the invention.
Fig. 1 is a Scanning Electron Microscope (SEM) image at 4000x magnification of the poly(vinylidene fluoride-co-hexafluoropropylene) copolymer containing MgO nanoparticles formed in a method of the disclosed embodiment.
Fig. 2 is a graph illustrating the adsorptive decomposition of: (a) paraoxon on polysulfone only; (b) paraoxon on PVDF copolymer only; (c) paraoxon on polysulfone with MgO (d) paraoxon on PVC with MgO; and (e) paraoxon on PVDF copolymer with MgO.
Fig. 3 is a graph illustrating the adsorption of (a) paraoxon on polysulfone only; (b) paraoxon on polysulfone with MgO; (c) paraoxon on MgO particle only; (d) paraoxon on activated charcoal particles only; (e) paraoxon on alumina particles only; and (f) paraoxon on polysulfone with Al₂O₃.

### Examples

The invention will be further described in greater detail by reference to specific Examples, which should not be construed as in any way limiting the scope of the invention.

### Ref. Example 1 - Aerogel method of preparing Magnesium Oxide nanoparticles

Magnesium methoxide was prepared by stirring magnesium and methanol (Merck & Co. Inc., New Jersey) at room temperature in the presence of nitrogen to form an inert atmosphere. After overnight stirring, the gel was autoclave heated to 250°C with 1°C/min heating rate and kept at this temperature for 15 minutes. The resulting white powder was subjected to further heat treatment using a modified procedure. The white powder was taken in a porcelain crucible and heated from room temperature to 220°C at 1°C/min and held at 220°C for 5 h. The temperature of the autoclave was again raised from 220°C to 400°C at 1°C/min and held at this temperature for 4 hours. The surface area of the synthesized MgO material was found to be 155 m²/g by BET method which is lower than reported (Utamapanya et al., Chem. Mater., 3, 175-181) (400m²/g)_{.}

### Ref. Example 2 - Method for preparing nanosized polymer fiber with embedded metaloxide nanoparticles

Polymer solutions of polysulfone polymer (PSU), poly[(vinylidene fluoride)-co-(hexafluoropropylene)] (PVDF copolymer), poly(vinyl chloride) (PVC) having different weight loadings of MgO nanoparticles (or Al₂O₃ nanoparticles) were prepared by mixing varying amount of MgO nanoparticles (or Al₂O₃ nanoparticles) and polymer solution. Commercially available nano-Al₂O₃ was purchased from Aldrich and after sieving of this material, surface area is found to be 280 m²/g by BET method.

PSU, PVDF copolymer, and PVC polymer used in the experiment respectively had a number average molecular weight of (Mₙ) 26,000, 110,000 and 99,000.

A typical procedure adopted for the preparation of PSU electrospun membrane is provided here. 2g of polymer and 8g of dimethyl formamide (DMF) were stirred magnetically at room temperature for about 12 h to dissolve the polymer. After complete dissolution of polymer, 200 mg of MgO nanoparticles was added and stirred for 24 hours at room temperature (about 22 deg. C). To achieve uniform dispersion of polymer solution, the mixture was sonicated. The resulting polymer solution was then loaded into a syringe fitted to a syringe pump. The positive terminal of a high voltage DC power supply was connected to the metallic needle of the syringe. A grounded aluminum foil placed 15 cm from the tip of the needle was used as the target to collect the membranes. The syringe pump was set to deliver the solution at a rate of 4 mL/hr and high voltage (12.5 KV) was applied. Electrospinning was carried out at room temperature in air with a relative humidity below 50%. The electrospun membrane was dried in a vacuum pump and tested for hydrolytic efficiency studies.

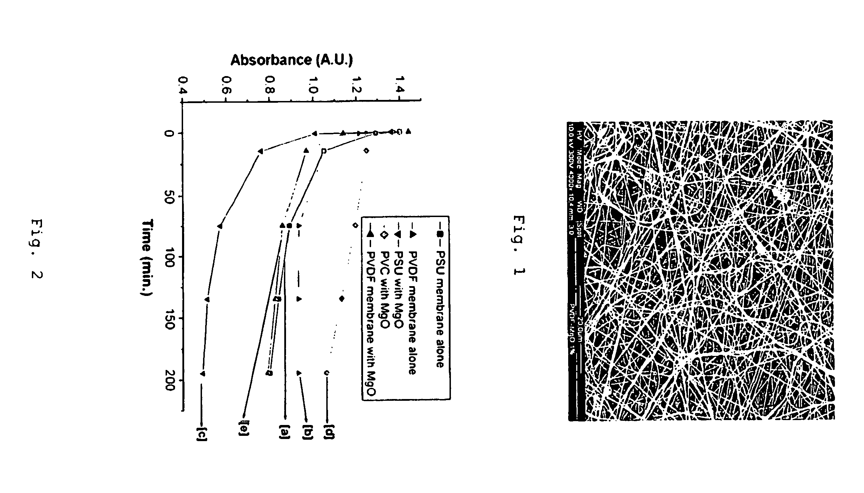

Fig. 1 is an SEM image at 4000x magnification of the nanocomposite membrane made up of polyvinylidene fluoride-co-hexafluoropropylene nanofiber containing MgO nanoparticles.

### Ref. Example 3 - Detoxification of paraoxon using nanocomposite fiber

After successful fabrication of nanocomposite membranes as described above, for a proof of concept and to select a suitable membrane, testing of detoxification of nerve stimulant paraoxon was carried out. UV studies were carried out for individual polymer membranes and membranes with MgO nanoparticles (refer to Fig. 2): (a) paraoxon on polysulfone only; (b) paraoxon on PVDF copolymer only; (c) paraoxon on polysulfone with MgO (d) paraoxon on PVC with MgO; and (e) paraoxon on PVDF copolymer with MgO.

Polysulfone was found to have superior adsorption properties compared to the PVDF (e) and PVC (d). These results suggest that the selection of an appropriate polymer support is also important in order to retain the MgO activity.

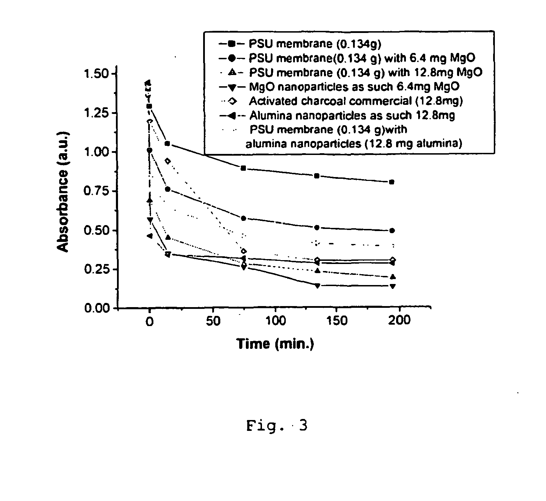

The UV adsorption study was also performed for paraoxon in heptane solution in presence of polysulfone polymer alone, nanocomposite membrane and MgO nanoparticles, Al₂O₃ nanoparticles, and charcoal with time (Fig. 3): (a) paraoxon on polysulfone only; (b) paraoxon on polysulfone with MgO; (c) paraoxon on MgO particle only; (d) paraoxon on activated charcoal particles only; (e) paraoxon on alumina particles only; and (f) paraoxon on polysulfone with Al₂O₃.

The obtained results indicated that the nanocomposite membrane has the capability of detoxifying a toxic agent without much loss of activity of MgO particles. The nanocomposite membrane made up of MgO is found to be more active than commercial activated charcoal which is used in canister application.

### Applications

Nanosized or micro-sized fiber comprising particles produced by an embodiment described herein can be used for a variety of purposes such as, but not limited to, a material for the removal of spillage on surfaces, protective clothing, filtration media or gas separation.

Advantageously, the fiber is lightweight, low cost, and highly effective in decontaminating/detoxifying chemical and biological agents.

Advantageously, the fiber decontaminates or detoxifies chemical and biological agents without producing any toxic by-products.

Advantageously, the fiber is breathable such that it can be used as protective garments or as a facemask. The nanocomposite membrane will allow air to pass through and adsorb chemical and biological agents selectively and the presence of pores will help enhanced adsorption. More advantageously, the particles are not airborne because they are bound to the fibers. Thus allowing the fiber to be used by (a) soldiers in battlefield (against warfare agents), (b) the general public (against viruses like avian flu, ebola, SARS), (c) industrial workers (against toxic chemical fumes or spillage), and (d) medical personnel (against highly infectious diseases).

Advantageously, the flexible nature of the fiber enables the fiber to attain any shapes and hence can be used as wipe cloths to contain and clean the spillage of chemical or biological agents on intricate parts of human body and other surfaces such as floors, soldier's equipments, transports, and costly instruments.

## Claims

1. A nano-sized or micro-sized fiber comprising detoxifying particles capable of at least partially detoxifying a toxic agent, said particles comprising metal oxide halogen adducts.

2. The fiber as claimed in claim 1, wherein the detoxifying particles are nano-sized or micro-sized.

3. The fiber as claimed in claim 1, wherein the fiber comprises a polymer, and optionally the polymer is an adsorbent to the toxic agent.

4. The fiber as claimed in claim 3, wherein the adsorbent polymer is selected from the group consisting of polysulfones, polyurethanes, polycarbonates and combinations thereof.

5. The fiber as claimed in claim 3, wherein the polymer fiber is electrospun from a spinning solution.

6. The fiber as claimed in claim 1, wherein the detoxifying particles comprise metal oxides selected from the group consisting of MgO, CaO, BaO, TiO₂, SrO, ZnO, Mn₂O₃, Fe₂O₃, FeO, ZrO₂, V₂O₃, V₂O₅, CuO, NiO, Al₂O₃, CeO₂, SnO₂, Fe₃O₄, MnO, Cu₂O, Nb₂O₅, InO₃, HfO₂, Cr₂O₃, Ta₂O₅, Ga₂O₃, Y₂O₃, MoO₃, Co₃O₄, SiO₂, ZnO, Ag₂O, Ag, and MgO/Al₂O₃.

7. The fiber as claimed in claim 6, wherein the halogen is selected from the group consisting of chlorine, iodine and bromine.

8. The fiber as claimed in claim 5, wherein the spinning solution comprises said detoxifying particles.

9. A method for producing a fiber comprising the step of electrospinning a spinning solution having a plurality of detoxifying particles suspended therein to form a nano-sized or micro-sized fiber comprising said detoxifying particles, wherein said detoxifying particles comprise metal oxide halogen adducts.

10. The method as claimed in claim 9, wherein the detoxifying particles are nano-sized or micro-sized.

11. The method as claimed in claim 9, wherein the detoxifying particles comprise metal oxides selected from the group consisting of MgO, CaO, BaO, TiO₂, SrO, ZnO, Mn₂O₃, Fe₂O₃, FeO, ZrO₂, V₂O₃, V₂O₅, CuO, NiO, Al₂O₃, CeO₂, SnO₂, Fe₃O₄, MnO, Cu₂O, Nb₂O₅, InO₃, HfO₂, ^{C} Cr₂O₃, Ta₂O₅, Ga₂O₃, Y₂O₃, MoO₃, Co₃O₄, SiO₂, ZnO, Ag₂O, Ag, and MgO/Al₂O₃.

12. The method as claimed in claim 11, wherein said halogen is selected from the group consisting of chlorine, iodine and bromine.

13. The method as claimed in claim 9, wherein the detoxifying particles are suspended in said spinning solution during said electrospinning step, and optionally the spinning solution comprises a polymer solution.

14. An article for detoxifying a toxic agent comprising a plurality of coupled nano-sized or micro-sized fibers, each of said fibers comprising detoxifying particles, wherein said particles comprise metal oxide halogen adducts.

15. An article as claimed in claim 14, wherein said article is a membrane of said fibers.

16. Use of the article of claim 14, in the detoxification of a toxic agent.

## Patentansprüche

1. Faser in Nanogröße oder Mikrogröße umfassend entgiftende Partikel welche imstande sind einen Giftstoff zumindest teilweise zu entgiften, wobei die Partikel Metalloxid-Halogen Addukte umfassen.

2. Faser gemäß Anspruch 1, wobei die entgiftenden Partikel Nanogröße oder Mikrogröße aufweisen.

3. Faser gemäß Anspruch 1, wobei die Faser ein Polymer umfasst, und gegebenenfalls das Polymer ein Adsorber des Giftstoffes ist.

4. Faser gemäß Anspruch 3, wobei das Adsorberpolymer ausgewählt ist aus der Gruppe bestehend aus Polysulfonen, Polyurethanen, Polycarbonaten und Kombinationen davon.

5. Faser gemäß Anspruch 3, wobei die Polymerfaser aus einer Spinnlösung elektrogesponnen ist.

6. Faser gemäß Anspruch 1, wobei die entgiftenden Partikel Metalloxide umfassen ausgewählt aus der Gruppe bestehend aus MgO, CaO, BaO, TiO₂, SrO, ZnO, Mn₂O₃, Fe₂O₃, FeO, ZrO₂, V₂O₃, V₂O₅, CuO, NiO, Al₂O₃, CeO₂, SnO₂, Fe₃O₄, MnO, Cu₂O, Nb₂O₅, InO₃, HfO₂, Cr₂O₃, Ta₂O₅, Ga₂O₃, Y₂O₃, MoO₃, Co₃O₄, SiO₂, ZnO, Ag₂O, Ag, und MgO/Al₂O₃.

7. Faser gemäß Anspruch 6, wobei das Halogen ausgewählt ist aus einer Gruppe bestehend aus Chlor, Iod und Brom.

8. Faser gemäß Anspruch 5, wobei die Spinnlösung die entgiftenden Partikel umfasst.

9. Verfahren zur Herstellung einer Faser umfassend den Schritt Elektrospinnen einer Spinnlösung, welche eine Vielzahl von entgiftenden Partikeln darin supendiert aufweist, um eine Faser in Nanogröße oder Mikrogröße zu bilden umfassend die entgiftenden Partikel, wobei die entgiftenden Partikel Metalloxid-Halogen Addukte umfassen.

10. Verfahren gemäß Anspruch 9, wobei die entgiftenden Partikel Nanogröße oder Mikrogröße aufweisen.

11. Verfahren gemäß Anspruch 9, wobei die entgiftenden Partikel Metalloxide umfassen ausgewählt aus der Gruppe bestehend aus MgO, CaO, BaO, TiO₂, SrO, ZnO, Mn₂O₃, Fe₂O₃, FeO, ZrO₂, V₂O₃, V₂O₅, CuO, NiO, Al₂O₃, CeO₂, SnO₂, Fe₃O₄, MnO, Cu₂O, Nb₂O₅, InO₃, HfO₂, Cr₂O₃, Ta₂O₅, Ga₂O₃, Y₂O₃, MoO₃, Co₃O₄, SiO₂, ZnO, Ag₂O, Ag, und MgO/Al₂O₃.

12. Verfahren gemäß Anspruch 11, wobei das Halogen ausgewählt ist aus einer Gruppe bestehend aus Chlor, Iod und Brom.

13. Verfahren gemäß Anspruch 9, wobei die entgiftenden Partikel in der Spinnlösung suspendiert sind während dem Elektrospinnen-Schritt, und gegebenenfalls die Spinnlösung eine Polymerlösung umfasst.

14. Gegenstand für entgiften eines Giftstoffes umfassend einen Vielzahl von gekuppelten Fasern in Nanogröße oder Mikrogröße, wobei jede der Fasern entgiftende Partikel umfasst, wobei die Partikel Metalloxid-Halogen Addukte umfassen.

15. Gegenstand gemäß Anspruch 14, wobei der Gegenstand eine Membran von besagten Fasern ist.

16. Verwendung von dem Gegenstand von Anspruch 14, in der Entgiftung von einen Giftstoff

## Revendications

1. Fibre d'une taille de l'ordre du nanomètre ou du micromètre comprenant des particules de décontamination capables de décontaminer au moins partiellement un agent toxique, lesdites particules comprenant des adduits d'un oxyde métallique et d'un halogène.

2. Fibre selon la revendication 1, dans laquelle les particules de décontamination ont une taille de l'ordre du nanomètre ou du micromètre.

3. Fibre selon la revendication 1, dans laquelle la fibre comprend un polymère et éventuellement le polymère est un absorbant de l'agent toxique.

4. Fibre selon la revendication 3, dans laquelle le polymère absorbant est choisi dans le groupe constitué par les polysulfones, les polyuréthanes, les polycarbonates et les combinaisons de ceux-ci.

5. Fibre selon la revendication 3, dans laquelle la fibre de polymère est filée par voie électrolytique à partir d'une solution de filage.

6. Fibre selon la revendication 1, dans laquelle les particules de décontamination comprennent des oxydes métalliques choisis dans le groupe constitué par MgO, CaO, BaO, TiO₂, SrO, ZnO, Mn₂O₃, Fe₂O₃, FeO, ZrO₂, V₂O₃, V₂O₅, CuO, NiO, Al₂O₃, CeO₂, SnO₂, Fe₃O₄, MnO, Cu₂O, Nb₂O₅, InO₃, HfO₂, Cr₂O₃, Ta₂O₅, Ga₂O₃, Y₂O₃, MoO₃, Co₃O₄, SiO₂, ZnO, Ag₂O, Ag et MgO/Al₂O₃.

7. Fibre selon la revendication 6, dans laquelle l'halogène est choisi dans le groupe constitué par le chlore, l'iode et le brome.

8. Fibre selon la revendication 5, dans laquelle la solution de filage comprend lesdites particules de décontamination.

9. Procédé de production d'une fibre comprenant l'étape de filage électrolytique d'une solution de filage comprenant une pluralité de particules de décontamination en suspension dans celle-ci, pour former une fibre d'une taille de l'ordre du nanomètre ou du micromètre comprenant lesdites particules de décontamination, dans lequel lesdites particules de décontamination comprennent des adduits d'un oxyde métallique et d'un halogène.

10. Procédé selon la revendication 9, dans lequel les particules de décontamination ont une taille de l'ordre du nanomètre ou du micromètre.

11. Procédé selon la revendication 9, dans lequel les particules de décontamination comprennent des oxydes métalliques choisis dans le groupe constitué par MgO, CaO, BaO, TiO₂, SrO, ZnO, Mn₂O₃, Fe₂O₃, FeO, ZrO₂, V₂O₃, V₂O₅, CuO, NiO, Al₂O₃, CeO₂, SnO₂, Fe₃O₄, MnO, Cu₂O, Nb₂O₅, InO₃, HfO₂, Cr₂O₃, Ta₂O₅, Ga₂O₃, Y₂O₃, MoO₃, Co₃O₄, SiO₂, ZnO, Ag₂O, Ag et MgO/Al₂O₃.

12. Procédé selon la revendication 11, dans lequel ledit halogène est choisi dans le groupe constitué par le chlore, l'iode et le brome.

13. Procédé selon la revendication 9, dans lequel les particules de décontamination sont en suspension dans ladite solution de filage pendant ladite étape de filage électrolytique, et éventuellement la solution de filage comprend une solution de polymère.

14. Article de décontamination d'un agent toxique comprenant une pluralité de fibres couplées d'une taille de l'ordre du nanomètre ou du micromètre, chacune desdites fibres comprenant des particules de décontamination, dans lequel lesdites particules comprennent des adduits d'un oxyde métallique et d'un halogène.

15. Article selon la revendication 14, dans lequel ledit article est une membrane desdites fibres.

16. Utilisation de l'article de la revendication 14, dans la décontamination d'un agent toxique.
